# EUROPEAN PATENT APPLICATION

(11) **EP 1 616 951 A1**
(43) Date of publication of application: **18.01.2006**
(21) Application number: 04728955.8
(22) Date of filing: 22.04.2004
(51) Int. Cl.: C12N 15/10, C12M 1/00, C12Q 1/68

(54) **METHOD OF ISOLATING NUCLEIC ACID AND, FOR NUCLEIC ACID ISOLATION, KIT AND APPARATUS**

(30) Priority: 22.04.2003 JP 2003116916
(71) Applicant: Arkray, Inc., Kyoto-shi, Kyoto 601-8045 (JP)
(72) Inventor: INOSE, Ken, ARKRAY, Inc., Kyoto-shi, Kyoto 601-8045 (JP); HASHIGUCHI, Satoshi, ARKRAY, Inc., Kyoto-shi, Kyoto 601-8045 (JP); IZUMIZAWA, Yuji, ARKRAY, Inc., Kyoto-shi, Kyoto 601-8045 (JP)
(74) Representative: Elsy, David
(86) International application number: PCT/JP2004/005811
(87) International publication number: WO 2004/094634

(57) **Abstract**

Nucleic acids suitable for PCR amplification are isolated from a sample easily and rapidly by dissolving the sample in a buffer containing surfactant and salt, heating the obtained solution, subjecting the heated solution to gel filtration; and collecting a fraction containing nucleic acids.

SEQUENCE LISTING

<110> ARKRAY, Inc.

<120> METHOD FOR ISOLATING NUCLEIC ACIDS, AND A KIT AND APPARATUS FOR NUCLEIC
ACID ISOLATION

<130> G872-OPC4071

<150> JP 2003-116916
<151> 2003-04-22

<160> 3

<170> PatentIn version 3.1

<210> 1
<211> 262
<212> DNA
<213> Homo sapiens

<400> 1
ggttggccaa tctactccca ggagcaggga gggcaggagc cagggctggg cataaaagtc 60

agggcagagc catctattgc ttacatttgc ttctgacaca actgtgttca ctagcaacct 120

caaacagaca ccatggtgca cctgactcct gaggagaagt ctgccgttac tgccctgtgg 180

ggcaaggtga acgtggatga agttggtggt gaggccctgg gcaggttggt atcaaggtta 240

caagacaggt ttaaggagac ca 262

<210> 2
<211> 22
<212> DNA
<213> Artificial

<220>
<223> primer

<400> 2
ggttggccaa tctactccca gg 22

<210> 3
<211> 22
<212> DNA
<213> Artificial

<220>
<223> primer

<400> 3
tggtctcctt aaacctgtct tg 22

## Description

### Technical Field

The present invention relates to a method for isolating nucleic acids from a sample and a kit and apparatus for nucleic acid isolation. The nucleic acids isolated by the method of the present invention are suitably used as a template for PCR.

### Background Art

To exactly detect a biological analyte that exists in various types of samples is necessary for many purposes including clinical, experimental, and epidemiological analysis. Most of the genetic information of every organism is delivered by deoxyribonucleic acids (DNA) and ribonucleic acids (RNA). Therefore, whether a specific analyte exists in a test sample or not can be determined by detecting and identifying a specific nucleic acid sequence.

The detection and identification of a nucleic acid having a specific sequence has become easy owing to the development of a polymerase chain reaction (PCR) that can amplify one or multiple target sequences in nucleic acids or a mixture thereof (US 4,965,188). In the PCR method, two kinds of primers which are substantially complementary to a portion of a target nucleic acid sequence to be amplified are designed and used. These primers are elongated by a thermostable enzyme to generate primer elongation products. When the primer elongation product is dissociated into single strands, each of the single strands further generates a template strand to be used for amplification of the target nucleic acid sequence. A primer binds to the template strand and are elongated by a thermostable enzyme, thereby, the same sequence as the target nucleic acid is synthesized and serves as a template. The PCR method involves an amplifying process by thermal cycles in which hybridization between a primer and a template and the synthesis of a primer elongation product by a polymerase are repeated depending on the thermal changes. The amount of the synthesized target nucleic acids increases exponentially by each cycle.

PCR amplification is useful in many clinical applications including the detection or diagnosis of infectious diseases, pathological chromosome aberrations, and DNA polymorphisms which may not relate to pathological states. PCR amplification is useful in such cases when a target nucleic acid exists in a smaller amount compared to other nucleic acid in a sample; only a small amount of a nucleic acid-containing sample is available; and rapid detection is desired. Specific examples of useful diagnosis application include diagnosis of hereditary diseases such as drepanocytic anemia, α-thalassemia, β-thalassemia, and pancreatic cystic fibrosis.

PCR method is applied as a useful method as described above, however, it is necessary to extract a nucleic acid mixture as an analyte from a sample and to purify it to a level to be used as a template in PCR. Examples of a method for extracting and purifying nucleic acids from a sample which has been used so far include a method in which a sample is dissolved in a buffer and proteins contained therein are removed with phenol/chloroform followed by precipitating nucleic acids with an alcohol such as ethanol (Molecular Cloning, A laboratory manual, 2nd ed., 1989, 3, pp. E3-E4), and a method in which a sample is dissolved in a buffer containing a chaotropic substance, and subjected to centrifugation to thereby obtain a supernatant, and the supernatant is adsorbed to a silica gel or the like, and after washing, nucleic acids are eluted (EP 389,063 A and JP2000-342259A). However, the former method has a problem that an organic solvent such as phenol should be handled carefully, and the latter method has such problems as contamination of a cleaning liquid and a low yield due to the repeated washing operations. Furthermore, both of the methods need many operations such as repeated centrifugation, which causes a problem that isolation of nucleic acids takes long time.

Meanwhile, a method has also been known, which involves mixing a sample with a buffer having a unique composition, centrifuging the mixture to obtain a supernatant, heating the supernatant, centrifuging the heat-treated solution to precipitate proteins, and subjecting the resulting supernatant to isopropanol precipitation to thereby precipitate nucleic acids (JP09-313181A). However, this method also takes long time because centrifugation operations should be repeated after the dissolution, after the heating, and after the addition of isopropanol. Furthermore, impurities cannot be completely removed with isopropanol precipitation, therefore this method is not always suitably used as a method for isolating nucleic acids for PCR amplification.

Purification of nucleic acids by a gel filtration method has been conventionally performed. However, the gel filtration method is mainly used for purification of PCR amplified products after completion of PCR procedures, and has never been used in a process of isolating nucleic acids from a biological sample.

### SUMMARY OF THE INVENTION

An object of the present invention is to provide a method for isolating nucleic acids from a sample easily, rapidly, and in high yield, and a kit and apparatus for nucleic acid isolation that can be used in such a method.

To achieve the above-mentioned object, the inventors of the present invention have made extensive studies. As a result, they found that DNAs from which PCR inhibitory substances are removed can be obtained rapidly and in high yield by dissolving a biological sample in a buffer containing surfactant and salt; heating the solution; and subjecting the heated solution to gel filtration, and thus completed the present invention

The present invention provides the followings.
(1) A method for isolating' nucleic acids from a sample containing nucleic acids comprising dissolving the sample in a buffer containing surfactant and salt; heating the obtained solution; subjecting the heated solution to gel filtration; and collecting a fraction containing nucleic acids.
(2) The method according to (1), wherein said surfactant is Triton X-100 (Registered Trademark).
(3) The method according to (1) or (2), wherein said salt is NaCl.
(4) The method according to any one of (1) to (3), wherein said sample is a sample containing eucaryotic cells.
(5) The method according to any one of (1) to (4), wherein said sample is blood.
(6) A kit for nucleic acid isolation from a sample containing nucleic acids, comprising a buffer and a gel filtration column, wherein said buffer contains at least one kind of surfactants and at least one kind of salts.
(7) The kit according to (6), wherein said buffer is a buffer containing Triton X-100 (Registered Trademark) and NaCl.
(8) An apparatus for nucleic acid isolation equipped with a sample-introducing part; a buffer-supplying part that supplies a buffer containing surfactant and salt; a heating part; and a separation part filled with gel filtration resins.

### BRIEF DESCRIPTION OF THE DRAWINGS

Fig. 1 (photograph) shows the results of PCR using as a template the ten-fold serial dilutions of the DNA solution obtained by QIAamp DNA Mini Kit. (A), (B), and (C) represent the results of PCR using the DNA solutions diluted in 1/10, 1/100, and 1/1,000, respectively. M represents a molecular weight marker (100bp ladder) and the number of each well represents a specimen number.
Fig. 2 (photograph) shows the results of PCR in which a half (12.5 µl) of the DNA solution obtained by QIAamp DNA Mini Kit is added to a reaction system. M represents a molecular weight marker (100bp ladder) and the number of each well represents a specimen number.
Fig. 3 (photograph) shows the results of PCR using as a template the ten-fold serial dilutions of the DNA solution obtained by GFX Genome Blood DNA Purification Kit. M represents a molecular weight marker (100bp ladder) and the number of each well represents a specimen number. (A), (B), and (C) represent the results of PCR using the DNA solutions diluted in 1/10, 1/100, and 1/1,000, respectively.
Fig. 4 (photograph) shows the results of PCR in which a half (12.5 µl) of the DNA solution obtained by GFX Genome Blood DNA Purification Kit is added to a reaction system. M represents a molecular weight marker (100bp ladder) and the number of each well represents a specimen number.
Fig. 5(photograph) shows the results of PCR using as a template the ten-fold serial dilutions of the DNA solution obtained by the isolation method of the present invention. M represents a molecular weight marker (100bp ladder) and the number of each well represents a specimen number. (A) and (B) represent the results of PCR using the DNA solutions diluted in 1/10 and 1/100, respectively.
Fig. 6 (photograph) shows the results of PCR in which a half of the DNA solution obtained by the isolation method of the present invention is added to a reaction system. M represents a molecular weight marker (100bp ladder) and the number of each well represents a specimen number.
Fig. 7 shows a schematic diagram of an apparatus for nucleic acid isolation of the present invention.

### DETAILED DESCRIPTION OF THE PREFERRED EMBODIMENTS

Hereinafter, the present invention will be explained in detail.

### <1> Method for isolating nucleic acids

The present invention relates to a method for isolating nucleic acids from a sample, comprising dissolving a sample in a buffer containing surfactant and salt; heating the solution; and subjecting the heated solution to gel filtration to obtain a fraction containing nucleic acids. Herein, the sample is not particularly limited as far as it contains nucleic acids, and examples thereof include various cell-containing samples to be used for gene analysis (e.g., PCR analysis). Preferable examples of the sample include a sample containing eucaryotic cells, such as blood, stool samples, cleaning fluid of oral or nasal cavity, soil, food, cultured cells, and microbial suspension. Among these, blood is particularly preferable.

The buffer for dissolving a sample is a buffer containing one or more kinds of surfactants and one or more kinds of salts. The buffer is not particularly limited, and examples thereof include phosphate buffer and Tris-EDTA buffer, and the Tris-EDTA buffer is preferable from the viewpoint of protection of nucleic acids. A specific example of the Tris-EDTA buffer includes, but not limited to, 10 mM Tris-1 mM EDTA solution which is generally used. The surfactant is not particularly limited, and polyethyleneglycol-mono-p-isooctylphenyl ether is preferable from the viewpoint of cytolysis and Triton X-100 (registered trademark) is particularly preferable. The surfactant concentration is preferably 0.1 to 5% and particularly preferably 0.3 to 1%. Kinds of the salts contained in the buffer are not particularly limited, and a salt of a monovalent cation is preferable because it loosens the binding of a DNA-binding protein, and NaCl is more preferable. In addition, the salt concentration is preferably 0.1 M to 5 M, and particularly preferably 0.5 M to 2 M.

In the present invention, at first, the buffer is added to a sample in such a manner that a ratio of the sample volume to the buffer volume becomes 1/3 to 1 /100 to dissolve the sample. To dissolve efficiently, it is preferable to mix the sample after addition of the buffer. The mixing may be performed using a vortex mixer IMS-1000 (TOKYO RIKAKIKAI CO., LTD.) or the like, or performed by hands. Mixing time is preferably 5 seconds or less, and more preferably 3 seconds or less for a more rapid operation. However, it may be longer depending on a property of a sample. The mixing operation is for lysing cell membranes contained in a sample, extracting nucleic acids from the cells, and loosening the binding of various DNA-binding proteins to nucleic acids.

Next, the obtained sample solution is heated. This operation denatures cell-derived proteins, particularly DNA-binding proteins. The heating can be performed using a well-known method or apparatus such as a water-bath or a dry block bath (e.g., one manufactured by Iuchi Seieido Co., Ltd.). Heating temperature is not particularly limited so long as it is a temperature at which a protein can be sufficiently denatured, and the temperature is preferably 80 to 100°C, more preferably 90 to 100°C, and particularly preferably 95 to 100°C. Heating time is not particularly limited so long as it is a time during which proteins can be sufficiently denatured under the heating condition, and preferably 3 to 15 minutes, more preferably 4 to 10 minutes, and particularly preferably 5 to 10 minutes.

Subsequently, a fraction containing nucleic acids is obtained by gel filtration of the heated sample solution. PCR inhibitory substances existing in the sample or cells are considered to have low molecular weights compared to nucleic acids, and many proteins aggregate and become insoluble through the heating process. Therefore, nucleic acids containing no PCR inhibitory substances can be efficiently purified from the sample by means of the gel filtration. In addition, the heated sample solution may be subjected to gel filtration directly, or may be subjected to gel filtration after centrifugation. Although the heated sample may be subjected to gel filtration after having been cooled down to around room temperature, the heated sample is preferably subjected to gel filtration without cooling. The resins used for gel filtration are not particularly limited. Examples thereof include resins generally used for a gel filtration operation such as Sephacryl S-400HR and Sephacryl S-500HR (both manufactured by Amersham Biosciences K.K.) and the resins contained in CHROMA SPIN-1000 (Clontech) and CENTRISPIN-40 (PRINCETON SEPARATIONS). The gel filtration operation can be performed, for example, by adding a sample after heating to a centrifugible tube which contains gel filtration resins, and centrifuging the tube at a low speed (e.g., 500 G or less) for a short time (e.g., 60 seconds or less). The centrifugation operation may be performed at room temperature or under cooling.

The nucleic acids isolated by the method of the present invention can be used, for example, for PCR using specific primers, and thereby genes that relate to diseases such as insulin-dependent diabetes, specific cancer, or the like can be detected. In addition, by PCR using primers specific to pathogenic bacteria such as infectious bacteria or food poisoning bacteria, each bacterial specie or bacterial genus can be specifically detected and identified. The nucleic acids obtained by the method of the present invention can also be used for hybridization with DNA chips and for construction of clone libraries.

### <2> Kit for nucleic acid isolation

The present invention also provides a kit for isolating a nucleic acid. The kit of the present invention is a kit including a buffer containing at least one kind of surfactants and at least one kind of salts and a gel filtration column. The buffer contained in the kit is one that can dissolve a sample, specifically a buffer containing such surfactant (e.g., Triton X-100) and salt (e.g., NaCl) as described above. Examples of the gel filtration column contained in the kit include a centrifugible spin column which is filled with the above-described gel filtration resins. The kit of the present invention may contain a reagent and primers for PCR in addition to the buffer and gel filtration column.

### <3> Apparatus for nucleic acid isolation

An apparatus for nucleic acid isolation of the present invention is an apparatus for nucleic acid isolation equipped with a sample-introducing part, an extraction buffer-supplying part that supplies an extraction buffer containing surfactant and salt; a heating part, and a separation part filled with gel filtration resins. Examples of the sample-introducing part include a sample-introducing part which has a component capable of setting a plate or a tube containing a sample, or a component for injection of a liquid sample such as blood. Examples of the extraction buffer-supplying part that supplies an extraction buffer containing surfactant and salt include a supplying part which has a container containing the buffer, and a supplying pump. Examples of the heating part include a heating part which has an electric heater or the like. The heating part is not necessarily set separately from the sample-introducing part, and the sample-introducing part itself may be heat-applicable part. Examples of the separation part filled with gel filtration resins include a separation part which has, for example, a column filled with gel filtration resins.

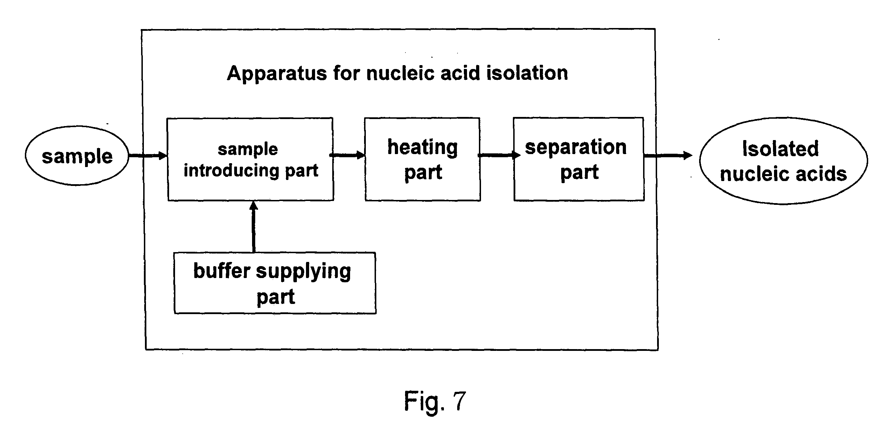

Fig. 7 shows a schematic view of the apparatus of the present invention. A sample set in a sample-introducing part is supplied with a buffer from a buffer-supplying part, and thereby the sample is dissolved in the buffer. The dissolved sample is heated in a heating part, and then separated in a separation part. A solution obtained after the separation in the separation part contains the isolated nucleic acids.

### Example

Hereinafter, the present invention will be described in detail with reference to the examples. However, the present invention is not limited to these examples.

### 1. DNA isolation

By the various methods as described below, DNAs were isolated from blood samples (containing an anticoagulant (heparin lithium)) of 10 normal adults, respectively. The isolated DNAs were subjected to PCR to thereby confirm the amount of DNA and to compare removal of PCR inhibitory substances.

### Comparative Example 1 (DNA isolation by the method using a buffer containing protease and silica gel column)

DNA was isolated from a blood sample using QIAamp DNA Mini Kit (QIAGEN). First, 200 µl of the blood sample was mixed with 20 µl of QIAGEN Protease and 200 µl of Buffer AL, both of which are attached to the kit. The mixture was mixed with vortex mixer for 15 seconds, followed by incubation at 56°C for 10 minutes. Next, after spin-down, 200 µl of 100% ethanol was added, followed by vortex-mixing for 15 seconds and spin-down. The mixture was poured into a silica gel spin column, which is attached to the kit, to bind DNAs and centrifuged at 6,000 g for 1 minute. 500 µl of the attached Buffer AW1 was added, followed by centrifugation at 6,000 g for 1 minute and washing. Then, 500 µl of the attached Buffer AW2 was added therein, followed by centrifugation at 20,000 g for 3 minutes and washing. Subsequently, the spin column was set into a new tube. 200 µl of the attached Buffer AE was added therein and left stand at room temperature for 1 minute, followed by centrifugation at 6,000 g for 1 minute, thereby eluting DNA extract solution. The time spent for the isolation was 25 minutes.

### Comparative Example 2 (DNA isolation by the method using a buffer containing chaotrope substance and silica gel column)

DNA was isolated from a blood sample using GFX Genome Blood DNA Purification Kit (Amersham Biosciences). 100 µl of a blood sample was mixed with 500 µl of the extraction solution (attached to the kit) containing chaotropic ions, and the mixture was mixed with vortex mixer and left stand for 5 minutes. The mixture was poured into the attached GFX column to bind DNAs and centrifuged at 5,000 g for 1 minute. 500 µl of the extraction solution was further added, followed by centrifugation at 5,000 g for 1 minute and washing. Subsequently, 500 µl of the attached wash solution was added, followed by centrifugation at 20,000 g for 1 minute and washing. The column was set into a new tube. 100 µl of the attached elution buffer pre-warmed to 70°C was added to the column, and left stand at room temperature for 1 minute, followed by centrifugation at 5,000 g for 1 minute, thereby DNA extract solution was obtained. The time spent for the isolation was 20 minutes.

### Example 1 (DNA isolation by the method of the present invention)

20 µl of blood was added with 180 µl of the extraction buffer (10 mM Tris-HCl (pH 8.0), 1 mM EDTA, 0.5% Triton X-100, 2 M NaCl) and suspended for 3 seconds, and the suspension was heated at 98C° for 5 minutes. During the heating, a gel filtration spin column was prepared. The gel filtration spin column was prepared by filling 600 µl of gel filtration resins (CHROMA SPIN-1000 (CLONTECH)) into the spin column (MicroSpin Empty Column (Amersham Biosciences)), centrifuging the spin column at 700 g for 2 minutes to remove a liquid therein. After heating, the sample was immediately mixed with a vortex mixer. Then, the sample was centrifuged at 500 G for 10 seconds, and 100 µl of the resulting supernatant was supplied to the gel filtration spin column prepared as described above. The spin column was centrifuged at 300 g for 1 minute, thereby eluted a DNA extract solution. The time spent for the isolation was 10 minutes.

### 2. PCR

Ten-fold serial dilutions (x 10, x 100, and x 1,000) of the prepared DNA solutions were prepared and used as a template in PCR in which β-globin (human) Primer Set (TAKARA BIO INC.) was used as primers to amplify β-globin. The reaction solution contained 10 mM Tris-HCl (pH 8.3), 50 mM KCI, 1.5 mM MgCl₂, 200 µM dNTP mixture, 0.5 µM of each of β-globin gene (SEQ ID No:1, amplified size of 262 bp)-specific primers (KM29 (SEQ ID NO:2) and KM38 (SEQ ID NO:3)), and 0.625 U Taq DNA Polymerase (TAKARA BIO INC.). 1 µl or 12.5 µl of the template DNA solution was added therein, and the total volume was adjusted to 25 µl by H₂O. The PCR program was as follows. That is, after heating at 94 °C for 1 minute, a cycle consisting of heating at 94 °C for 1 minute, 55 °C for 2 minutes and 72 °C for 1 minute was repeated 30 cycles, followed by heating at 72 °C for 5 minutes. 6 µl of the amplified product was analyzed by electrophoresis on 3% agarose gel and ethidium bromide staining.

### 3. Results

### 3-1. PCR using the DNAs isolated by QIAamp DNA Mini Kit (Comparative Example 1)

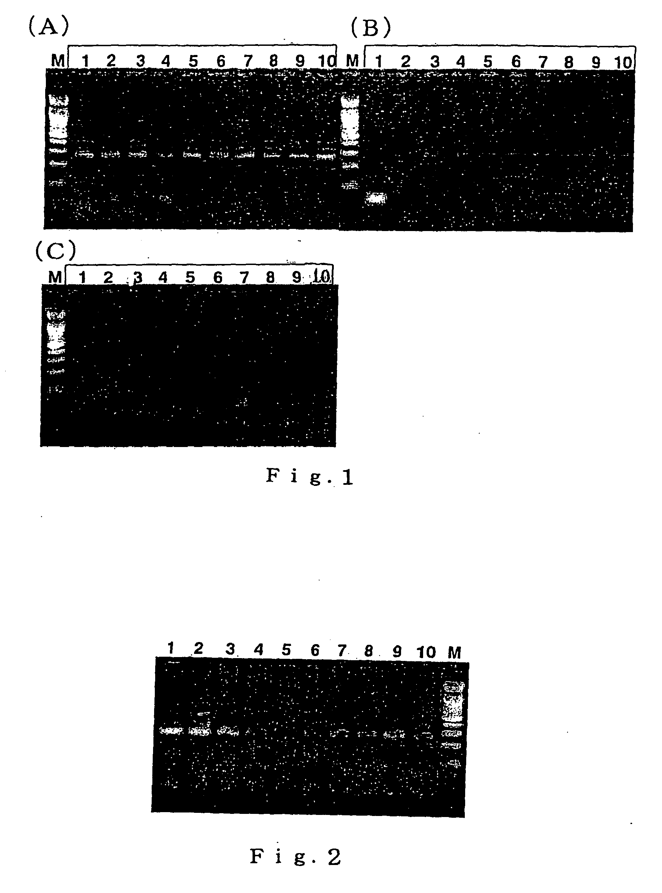

DNAs were extracted respectively from 10 samples using QIAamp DNA Mini Kit in the same manner as Comparative Example 1 and subjected to PCR to confirm the presence or absence of the objective DNA. As a result, the amplified product was observed in 9 samples, even in the samples diluted to 1 /100, although no amplified product was observed in the specimen number 1 (Fig. 1). On the other hand, when 12.5 µl of the extracted sample solution was added to the reaction system and PCR was carried out, no amplified product was observed in the specimen number 5. In the remaining 9 samples, amplified products were observed, but the amounts of the amplified products were small (Fig. 2). This suggested the contamination of PCR inhibitory substances.

### 3-2. PCR using DNA isolated by GFX Genome Blood DNA Purification Kit (Comparative Example 2)

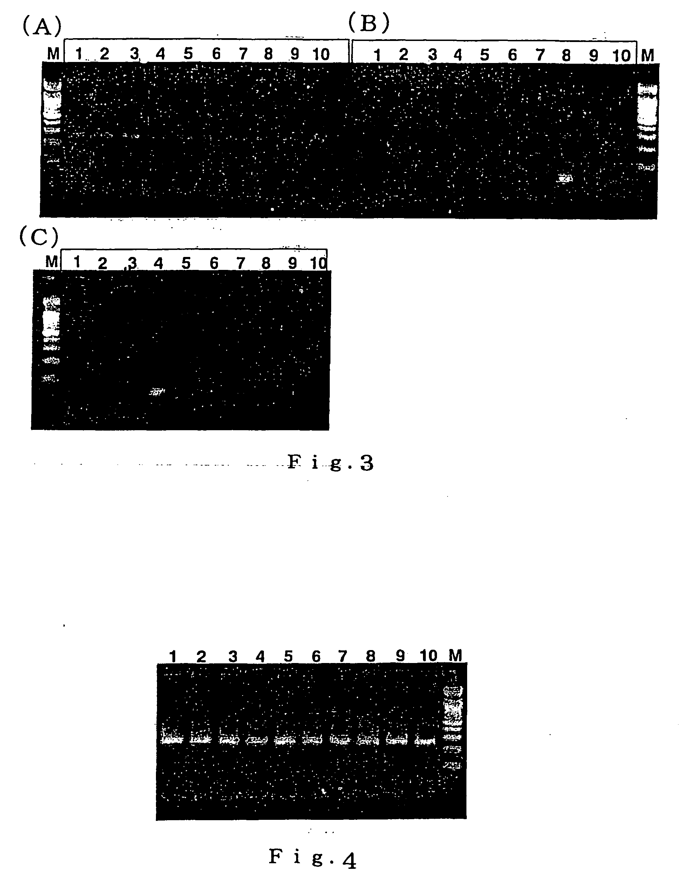

DNAs were extracted respectively from 10 samples using GFX Genome Blood DNA Purification Kit in the same manner as Comparative Example 2 and the presence or absence of DNA was confirmed by PCR. As a result, amplified products were observed in 6 samples, even in the samples diluted to 1/100, although no amplified product was observed in the specimen numbers 5, 6, 8, and 9 (Fig. 3). On the other hand, when 12.5 µl of the extracted sample solution was added to the reaction system and PCR was carried out, an amplified product was observed in every sample, which suggested that inhibitory substances have been removed (Fig. 4).

### 3-3. PCR using the DNAs isolated by the method of the present invention (Example 1)

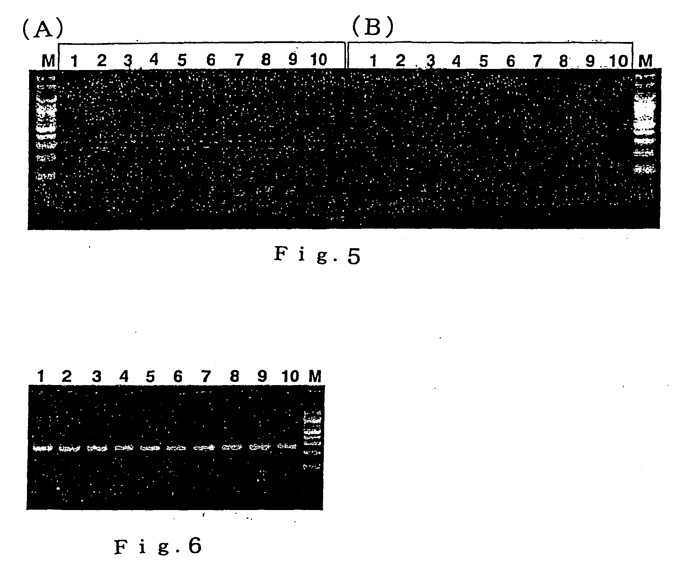

DNAs were extracted respectively from 10 samples by the isolation method of the present invention and the presence or absence of the objective DNA was confirmed by PCR. As a result, an amplified product was observed in every sample, even in the samples diluted to 1/100 (Fig. 5). Furthermore, when 12.5 µl of the extracted sample was added to the reaction system and PCR was carried out, an amplified product was observed in every sample, which confirmed that inhibitory substances were efficiently removed (Fig. 6).

As described above, in the case where the isolation method of the present invention was employed, an equal or larger amount of amplified DNA was obtained by PCR as compared to the other two methods, even though the amount of a starting sample (20 µl of blood) was fewer than that in the other two methods (Comparative Example 1: 200 µl, Comparative Example 2: 100 µl). That is, it was confirmed that DNAs can be extracted rapidly and in high yield and PCR inhibitory substances can be efficiently removed by the method of the present invention.

### Industrial Applicability

According to the method of the present invention, nucleic acids suitable for PCR can be isolated more rapidly and easily from a sample containing many contaminants such as PCR inhibitory substances in an yield equal to or larger than that of the conventional methods. By using the nucleic acids isolated by the method of the present invention, gene analysis using PCR or the like can be performed more rapidly than a conventional method.

## Claims

1. A method for isolating nucleic acids from a sample containing nucleic acids comprising:
dissolving the sample in a buffer containing surfactant and salt;
heating the obtained solution;
subjecting the heated solution to gel filtration; and
collecting a fraction containing nucleic acids.

2. The method according to claim 1, wherein said surfactant is Triton X-100 (Registered Trademark).

3. The method according to claim 1 or 2, wherein said salt is NaCl.

4. The method according to any one of claims 1 to 3, wherein said sample is a sample containing eucaryotic cells.

5. The method according to any one of claims 1 to 4, wherein said sample is blood.

6. A kit for nucleic acid isolation from a sample containing nucleic acids, comprising
a buffer and a gel filtration column, wherein said buffer contains at least one kind of surfactants and at least one kind of salts.

7. The kit according to claim 6, wherein said buffer is a buffer containing Triton X-100 (Registered Trademark) and NaCl.

8. An apparatus for nucleic acid isolation equipped with:
a sample-introducing part;
a buffer-supplying part that supplies a buffer containing surfactant and salt;
a heating part; and
a separation part filled with gel filtration resins.
